# EUROPEAN PATENT APPLICATION

(11) **EP 4 846 942 A1**
(43) Date of publication of application: **02.12.2026**
(21) Application number: 25179402.0
(22) Date of filing: 28.05.2025
(51) Int. Cl.: A61B 6/04, A61B 6/50, A61B 6/00, A61B 6/58

(54) **X-RAY IMAGING DURING HUMAN BRAIN RELATED INTERVENTIONS**

(71) Applicant: Koninklijke Philips N.V., 5656 AE Eindhoven (NL)
(72) Inventor: DRIES, Johan Juliana, Eindhoven (NL); VAN DER WACHT, Rogier Sean, Eindhoven (NL); GAST, Tomas, Eindhoven (NL); VAN HEESCH, Frank, Eindhoven (NL); HENDRIKS, Bernardus Hendrikus Wilhelmus, Eindhoven (NL); ELENBAAS, Thijs, Eindhoven (NL)
(74) Representative: Philips Intellectual Property & Standards

(57) **Abstract**

The present invention relates to X-ray imaging of the head. In order to provide improved images during human brain related interventions, a control device (10) for acquiring X-ray images during human brain related interventions is provided. The control device comprises a data input (12), a data processor (14) and an output interface (16). The data input is configured to provide sensor data (18) representing a 3D contour of a current situation, comprising a subject's head that is temporarily fixated with a headclamp. The data processor is configured to detect the headclamp in the 3D contour, and to compute a trajectory (20) for an X-ray imaging procedure of the head to provide X-ray image data comprising a sequence of at least two images of the subject's head from different directions. The data processor is configured to generate the trajectory such that artifacts due to the headclamp are minimized. The output interface is configured to provide the trajectory for X-ray imaging of the subject's head in the current situation.

## Description

### FIELD OF THE INVENTION

The present invention relates to X-ray imaging of the head, e.g. during brain interventions. The present invention relates in particular to a control device for acquiring X-ray images during human brain related interventions, to a medical X-ray imaging system and to a method for acquiring X-ray images during human brain related interventions.

### BACKGROUND OF THE INVENTION

X-ray imaging is used for providing information about areas of interest of a subject, e.g. areas inside the body. An example is the head region where X-ray is used in relation with neuro examinations, interventions and treatments. X-ray imaging can be used, for example, in the context of brain surgery. Imaging like CT provides 3D image data in form of so-called 3D reconstruction scans, which allows to generate a 3D model, which can then be used for generating projection images to be presented to the user. Not only during imaging, but in particular during interventions, headclamps can be used to ensure a fixated positioning of the subject's head. However, such headclamps can cause reconstruction artifacts which can be annoying, and which might also lead to medical misinterpretation.

### SUMMARY OF THE INVENTION

There may thus be a need to provide improved images during human brain related interventions.

The object of the present invention is solved by the subject-matter of the independent claims; further embodiments are incorporated in the dependent claims. It should be noted that the following described aspects of the invention apply also for the control device for acquiring X-ray images during human brain related interventions, for the medical X-ray imaging system and for the method for acquiring X-ray images during human brain related interventions.

According to the present invention, a control device for acquiring X-ray images during human brain related interventions is provided. The control device comprises a data input, a data processor and an output interface. The data input is configured to provide sensor data representing a 3D contour of a current situation, comprising a subject's head that is temporarily fixated with a headclamp. The data processor is configured to detect the headclamp in the 3D contour, and to compute a trajectory for an X-ray imaging procedure of the head to provide X-ray image data comprising a sequence of at least two images of the subject's head from different directions. The data processor is configured to generate the trajectory such that artifacts due to the headclamp are minimized. The output interface is configured to provide the trajectory for X-ray imaging of the subject's head in the current situation.

As an effect, a proposal of an optimized scan as part of the 3D-reconstruction workflow is provided.

As an advantage, the need to reposition the patient is avoided or at least the chances are significantly reduced. Avoiding repositioning also addresses aspects further on in the interventional procedure, as the initial positioning also addresses positioning the patient optimally for the surgery.

According to an example, the data processor is configured to detect the subject's head in the 3D contour, and to compute the trajectory also based on the detected subject's head.

This allows to further optimize the trajectory, since it also considers e.g. the size and shape of the subject's head.

According to an example, the data processor is configured to detect further body parts of the subject in the 3D contour that might interfere with X-ray imaging, and to compute the trajectory also based on the detected further body parts of the subject.

As an effect, the trajectory can be further adapted and therefore optimized to the respective situation.

According to an example, for detecting the subject's head or body parts or headclamp parts, the data processor is configured to provide one of the group comprising image recognition, contour reconstruction and pose tracking.

According to an example, for computing the trajectory, the data processor is configured to simulate possible changes for the arrangement of the headclamp and to consider the changes for computing alternative trajectories. The data processor is configured to compare the alternative trajectories and to select at least one and to provide the respective selection as proposal for a change of the headclamp.

According to the present invention, also a medical X-ray imaging system is provided. The imaging system comprises an X-ray imaging device with a movable X-ray source and a movable X-ray detector, a control device according to one of the preceding examples and a sensor arrangement. The sensor arrangement is configured to provide the sensor data representing a 3D contour of a current situation. The X-ray imaging device is configured to provide X-ray image data of a subject's head from different directions along the trajectory generated by the control device.

According to an example, the sensor arrangement comprises at least one sensor attached to the X-ray source or to the X-ray detector.

As an advantage, using the same support structure that already exists, any interference with the imaging procedure is avoided. The attachment to the moving parts of the imaging system allows to use the same mechanism to also move the sensors.

According to an example, the at least one sensor is an optical camera, a distance camera or a Lidar sensor.

These are relatively small and do not obstruct the view of the handling of the imaging apparatus.

According to an example, the X-ray imaging device is configured to perform a scanning movement with the sensor arrangement to provide a sensor scan of the current situation.

As an advantage, the scan movement allows to generate sensor data that covers all parts of the situation.

According to an example, the X-ray imaging device comprises a movable holding structure for the X-ray detector, and a separate movable holding structure for the X-ray source. As an option, the movable holding structure for the X-ray detector comprises a detector robot, and the movable holding structure for the X-ray source comprises a source robot.

This allows an optimized tracking and capturing of the present scenario.

According to an example, the sensor arrangement is configured to detect material properties of objects.

As an advantage, this option allows that also the respective effect on X-ray radiation can be determined and taken into account.

According to the present invention, also a method for acquiring X-ray images during human brain related interventions is provided. The method comprises the following steps:
- Generating sensor data representing a 3D contour of a current situation, comprising a subject's head that is temporarily fixated with a headclamp;
- Detecting the headclamp in the 3D contour;
- Computing, based on the detected headclamp, a trajectory for an X-ray imaging procedure of the head to provide X-ray image data of the subject's head from different directions; the trajectory is generated such that artifacts due to the headclamp are minimized; and
- Providing the trajectory for X-ray imaging of the subject's head in the current situation. According to an example of the method, it is provided the steps of:
- Clamping a subject's head in an initial position with a headclamp;
- Scanning the current situation to generate the sensor data representing the 3D contour;
- Simulating an X-ray scan and assessing the simulation results in terms of artifacts;
- Providing at least one proposal for a change of the headclamp position to minimize simulated artifacts;
- Selecting one of the at least one proposals for change and readjusting the headclamp;
- Scanning the resulting situation and computing an updated trajectory; and
- Providing the updated trajectory for acquiring a plurality of images.

By applying the computed trajectory for the imaging, it is avoided that the clamps produce artifacts, such as affected areas, affected transitions or streaks that would possibly obscure the brain anatomy in the image.

Even though the clamp may be of complex geometry, the computed trajectory results in "clean" image data, i.e. image data without artefacts, or minimal artifacts or at least with less artifacts.

According to an aspect, an optimal scan trajectory is provided that is based on a sensed 3D contour, which is currently present.

According to an aspect, a sensor is mounted next to the detector. If an imaging system without a C-arm, but with a source robot arm and a detector robot arm, is provided, the detector robot arm of such a system without a C-arm can be used to scan around the area or region of interest, creating a 3D contour that also contains the position of the headclamp. This sensor can e.g. be an optical camera, a hyperspectral camera, a distance camera or a small Lidar sensor. Based on the sensed 3D contour and knowledge about artifact causes, an optimal scan trajectory can be proposed and executed with the system without a C-arm.

As an example, in neuro surgical applications, an intervention is needed inside the human brain. Access is created by removing part of the skull. To avoid movement of the patient's head during the delicate surgical steps, a headclamp is used which fixes the position and orientation of the head. In order to improve the accuracy of the intervention, the surgeon wants to have a 3D-reconstruction of the brain after the skull removal, otherwise brain-shift affects a proper accuracy. Due to adapted trajectory, 3D-artifacts in the 3D-reconstruction volume caused by the headclamp are avoided or at least substantially reduced.

A field of application is mainly brain surgery application in hybrid operational rooms. Brain surgery is also referred to as neurosurgery.

In another aspect, a 3D-contour is provided via the sensors that comprises information about the shape and position of the headclamp. Further, as an option, also information about the material properties is provided. As an example, this is generated via shape comparison with a database of known headclamps including their material properties. This knowledge is used by a reconstruction procedure, e.g. a reconstruction software, for example by a compensation of the headclamp contributions in the acquired images, thus enhancing the result and reducing the presence of artifacts. As an intermediate result, this can be used to achieve improvements in addition to an adaptation of a scanning trajectory.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a control device for acquiring X-ray images during human brain related interventions.
Fig. 2a shows a first example of a situation during a human brain related intervention with a first example of a headclamp.
Fig. 2b shows a second example of a situation during a human brain related intervention with a second example of a headclamp.
Fig. 3 shows a first example of a medical X-ray imaging system.
Fig. 4 shows a second example of a medical X-ray imaging system.
Fig. 5 shows three different approaches of subject recognition.
Fig. 6 shows basic steps of an example of a method for acquiring X-ray images during human brain related interventions.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

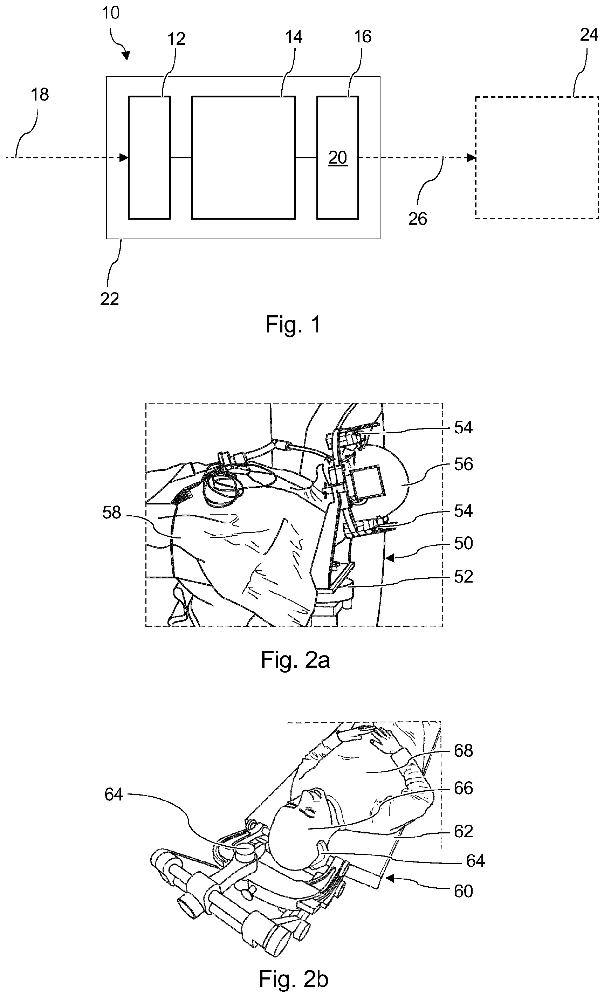

Fig. 1 schematically shows an example of a control device 10 for acquiring X-ray images during human brain related interventions. The control device 10 comprises a data input 12, a data processor 14 and an output interface 16. The data input 12 is configured to provide sensor data 18 representing a 3D contour of a current situation, comprising a subject's head that is temporarily fixated with a headclamp. The data processor 14 is configured to detect the headclamp in the 3D contour. The data processor 14 is also configured to compute a trajectory 20 for an X-ray imaging procedure of the head to provide X-ray image data comprising a sequence of at least two images of the subject's head from different directions; the data processor 14 is configured to generate the trajectory such that artifacts due to the headclamp are minimized. The output interface 16 is configured to provide the trajectory for X-ray imaging of the subject's head in the current situation.

The term "contour" relates to a spatial profile provided by different kind of objects within the field of view. As an example, the surface of a subject's head and the attached clamp provide the contour.

The term "sensor data" relates to data that provides information about the three-dimensional shape of the current situation, i.e. the location and form of the resulting surfaces.

In an example, the sensor data comprises depth information, e.g. information on which surfaces are having which distance to the sensor, i.e. which surface is where in the viewed space.

In an example, the sensor data is based on emitted electromagnetic rays or acoustic waves and their reflection on the surfaces forming the contour.

The computed trajectory can also be referred to as optimized trajectory, as adapted trajectory or as generated trajectory.

In an example, the sequence of images comprises many X-ray images of different projection angles. The images are X-ray images which contain information about the complete patient-volume where they have passes through.

The computed trajectory can also be referred to as adapted trajectory.

The term "trajectory" relates to a virtual path in space along which the imaging takes place. As an example, the trajectory defines the moving path for the imaging. As a further example, the trajectory also defines the spatial orientation during the movement along the path, i.e. the viewing direction.

In an option, the trajectory comprises a source trajectory and a detector trajectory.

In a variation, both trajectories are arranged around a common ISO-center. For example, the source and detector are mounted to the same rigid structure, like a C-arm.

In another variation, both trajectories are arranged as individual paths. For example, the source and detector are mounted to individual support structures, like two robot arms or two robots, or any other mechanical support arrangement that allows individual movement of the source in relation to the detector.

The headclamp is detected in the sensor data for example based on form or shape recognition, as the headclamp will always have characteristic edges that differ from the varying anatomic shapes and contours.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

The term "headclamp" refers to a surgery clamp used for holding the subject's head in place. Purpose of the clamp is to fix the position of the head during neurosurgery. The head is thus releasably and temporarily fixed by the clamp. The headclamp is also known as Mayfield clamp. In an example, the headclamp comprises two clamp arms to fixate the head. The data processor 14 is configured to detect both clamp arms in the 3D contour; and to generate the trajectory such that artifacts due to the two clamp arms are minimized.

In an example, the computed trajectory is provided as a non-circular scan. In an example, the computed trajectory is provided as a so-called dual-axes orbit. As an example, the computed trajectory performs a 3D scanning using a combined motion, i.e. moving trajectory that combines rotation, e.g. rotation around the subject's longitudinal axis and angulation, e.g. angulation towards head or feet of the subject, called "dual axes", instead of a circular scan, called "single axis".

As an example, the computed trajectory avoids that the headclamp is (too much / regularly) within the field of radiation during the imaging along the computed trajectory. As a further example, the computed trajectory also avoids that knobs of the clamp coincide in perspective.

The dual-axes scanning can be further modified. It is also possible that the computed trajectory provides so-called "complete" orbits.

In an example, the generated trajectory is provided as a non-circular and non-planar trajectory.

Fig. 2a shows a first example of a situation during a human brain related intervention. A first headclamp 50 is mounted to a support 52. The first headclamp 50 has a support structure 54 configured to temporarily fix a head 56 of a subject 58.

Fig. 2b shows a second example of a situation during a human brain related intervention. A second headclamp 60 is mounted to a support 62. The second headclamp 60 has a support structure 64 configured to temporarily fix a head 66 of a subject 68.

In both examples of the headclamps 50 and 60, the control device 10 is capable of computing a trajectory for an X-ray imaging procedure of the head to provide X-ray image data comprising a sequence of at least two images of the subject's head from different directions, in which images artifacts due to the headclamp are minimized.

In an option of the control device 10 in Fig. 1, the data processor 14 is configured to detect the subject's head in the 3D contour, and to compute the trajectory also based on the detected subject's head.

In an option of the control device 10 in Fig. 1, the data processor 14 is configured to detect further body parts of the subject in the 3D contour that might interfere with X-ray imaging, and to compute the trajectory also based on the detected further body parts of the subject.

In an option of the control device 10 in Fig. 1, for detecting the subject's head or body parts or headclamp parts, the data processor 14 is configured to provide one of the group comprising image recognition, contour reconstruction and pose tracking.

In an option of the control device 10 in Fig. 1, for computing the trajectory, the data processor 14 is configured to consider different views having a common volume that contains the region of interest.

In an option of the control device 10 in Fig. 1, for computing the trajectory, the data processor 14 is configured to simulate possible changes for the arrangement of the headclamp and to consider the changes for computing alternative trajectories. The data processor 14 is also configured to compare the alternative trajectories and to select at least one and to provide the respective selection as proposal for a change of the headclamp.

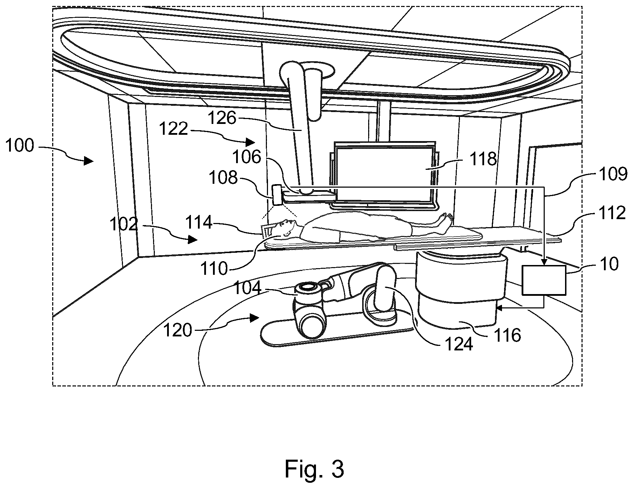

Fig. 3 shows a first example of a medical X-ray imaging system 100. The X-ray imaging system 100 comprises an X-ray imaging device 102 with a movable X-ray source 104 and a movable X-ray detector 106. The X-ray imaging system 100 further comprises an example of the control device 10 according to one of the preceding examples, and a sensor arrangement 108. The sensor arrangement 108 is configured to provide the sensor data representing a 3D contour of a current situation. The X-ray imaging device 102 is configured to provide X-ray image data of a subject's head from different directions along the trajectory generated by the control device 10.

A data connection line 109 is indicated to data-connect the sensor arrangement 208 with the control device 10. The connection can be wire-bound or wireless. The control device 10 is data-connected (not shown in Fig. 3) to the X-ray imaging device 102.

In Fig. 3, an object 110 is arranged on an object support 112. The object's head is fixated with a headclamp 114. The object support 112 is movably supported by a base 116. Further, a monitor arrangement 118 is provided.

In an option, shown in Fig. 3, the X-ray imaging device 102, 202 comprises a movable holding structure for the X-ray detector, and a separate movable holding structure for the X-ray source. As an option, the movable holding structure for the X-ray detector comprises a detector robot, and the movable holding structure for the X-ray source comprises a source robot.

In Fig. 3, the X-ray imaging device 102 is shown as an imaging system with two robots.

The X-ray source 104 is mounted to a first robot 120; the X-ray detector 106 is mounted to a second robot 122. The first robot 120 may comprise a robot arm arrangement 124 with two or more arms mounted to a floor-supported moveable base. The second robot 122 may comprise a further robot arm arrangement 126 with two or more arms mounted to a ceiling-suspended moveable base.

Such structure is a structure without a C-arm and can also be referred to as C-less structure.

The system without a C-arm X-ray gantry is very flexible in its movements, because two robotic arms are used to position the X-ray tube and the detector independently: It can create more distance between the center of rotation and the detector, reducing the risk of collision with the headclamp and greatly increasing the likelihood that the scan can actually be performed. It is also more flexible to make the 3D-scan under angles or even to change the OID (distance object to detector) or SID (distance between source and detector) during the scan if needed, e.g. to make space for needles or other surgical tools that are sticking out.

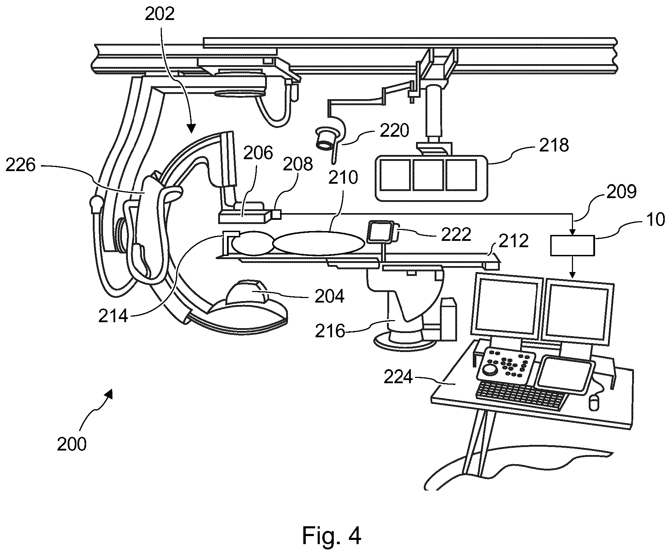

Fig. 4 shows a second example of the medical X-ray imaging system 200. The X-ray imaging system 200 comprises an X-ray imaging device 202 with a movable X-ray source 204 and a movable X-ray detector 206. The X-ray imaging system 200 further comprises an example of the control device 10 according to one of the preceding examples, and a sensor arrangement 208. The sensor arrangement 208 is configured to provide the sensor data representing a 3D contour of a current situation. The X-ray imaging device 202 is configured to provide X-ray image data of a subject's head from different directions along the trajectory generated by the control device.

A data connection line 209 is indicated to data-connect the sensor arrangement 208 with the control device 10. The connection can be wire-bound or wireless. The control device 10 is data-connected (not shown in Fig. 4) to the X-ray imaging device 202.

In Fig. 4, an object 210 is arranged on an object support 212. The object's head is fixated with a headclamp 214. The object support 212 is movably supported by a base 216. Further, a monitor arrangement 218 is provided. Further, lighting 220 is indicated. As an option, also a bedside control interface 222 is provided. An operating console 224 is shown in the right foreground. The console 224 comprises operating interfaces like a keyboard, mouse, touch pad, control knobs and displays.

In Fig. 4, the X-ray imaging device 102 is shown as a C-arm imaging system with a C-arm 226 movably mounted, e.g. to a ceiling-suspended rail system. The X-ray source 204 and the X-ray detector 206 are mounted to opposite arms of the C-arm 226.

In option, shown in Fig. 3 and Fig. 4, the sensor arrangement 108, 208 comprises at least one sensor attached to the X-ray detector 106, 206 or to the X-ray source 104, 204.

As an example, the at least one sensor is an optical camera.

As another example, the at least one sensor is a distance camera.

As a further example, the at least one sensor is a Lidar sensor.

In an option, a combination of any of these examples for a sensor is provided.

The sensor, i.e. the additional sensor to the X-ray imaging device, can be any sensor that provides data which can be used to generate 3D-contours. Some examples are: a distance camera (or a 3D-camera), a Lidar sensor, or an optical camera. Another example is a thermal camera or a hyperspectral camera. The sensor can be mounted next to the detector or on another component of the support structure like a robot.

In option, shown in Fig. 3 and Fig. 4, the X-ray imaging device 102, 104 is configured to perform a scanning movement with the sensor arrangement 108, 208 to provide a sensor scan of the current situation.

The optimized trajectory proposed by the system, based on the sensed 3D-contour of the patient including the headclamp, can prevent or decrease the occurrence of 3D-reconstruction artifacts. With this, optimized (non-circular) trajectories are feasible, that minimize absorption of the headclamp, optimize spatial resolution, while still resulting in complete filtered back projections. This ensures the surgeon that he can perform the right diagnosis and/or proceed the intervention with increased accuracy.

If the detector robot is equipped with an additional sensor, a scanning movement can be performed on a safe distance from the patient while reading this sensor. During this scanning movement, the tube robot can remain underneath the patient table, avoiding any issues from that side.

In an option, the 3D-contour of the patient's head, including the headclamp, is combined with knowledge on causes for 3D-reconstruction artifacts. As a result, an optimal trajectory for the X-ray gantry can be proposed that: reduces the fear for collisions by providing enough space between the critical elements and the moving detector and X-ray tube; results in optimized (non-circular) trajectories that minimize absorption of the headclamp, optimize spatial resolution, while still resulting in complete filtered back projections. This will prevent 3D-reconstruction artifacts or at least reduce the effect of the artifacts to an acceptable magnitude which cannot cause misinterpretation. This further optimizes the required X-ray dose by minimizing the distance between the detector and the center of rotation (and thus the X-source). The X-ray dose is also optimized by avoiding scans that do not deliver useful 3D-reconstruction data.

In an option, the sensor arrangement 208 is configured to detect material properties of objects.

The sensor is, for instance, a hyperspectral camera. Material properties determine the visibility in the X-ray image (and also what kind of artifacts they may cause). Taking these into account in determining the optimal trajectory of the X-ray source/detector, is of further advantage.

In an example, an additional material property sensor is provided, e.g. attached to the X-ray source or to the X-ray detector.

In another example, the same sensor used for detecting the 3D contour, also detects material properties. In an example, the combined sensor is attached to the X-ray source or to the X-ray detector.

In an example, the camera is a hyperspectral or multispectral camera allowing not only to determine the presence and shape of the objects in the room, such as the headclamp, but also to determine the material properties of the object parts. For instance, the headclamp is made of metal that can result in severe image artifacts, while when made of carbon the effect is less. Taking into account these material properties can help in determining the optimal scan trajectories.

Hyperspectral cameras can be of the type of push broom scanners, band sequential scanners or snapshot hyperspectral imagers. The snapshot hyperspectral camera can operate in real-time. These cameras cover wavelength ranges, such as visible spectrum (VIS), visible and near infrared (VNIR), near infrared (NIR), short-wavelength infrared (SWIR), mindware infrared (MWIR) and long-wave infrared (LWIR), allowing to detect various spectral features from objects that allow determining their material properties.

In an example, patient detection is provided. Detecting a contour of the patient can be challenging in the catheterization laboratory, since during the procedure a significant part of the patient may be covered with drapes. To solve this, a pose detection algorithms such as Alpha Pose or Open Pose is provided. These algorithms are trained to determine the stick figure of a person in the room, even when they are partly covered by drapes. These algorithms can partially be retrained to detect the stick figure of a patient lying on the table of the X-ray system that is partially draped. Knowing the stick figure of the patient, the shape of the person can be reconstructed using simple or even anatomical models.

The computing of a trajectory based on sensor data, which is non-X-ray data, provides the advantage that a 3D X-ray scan can be performed to provide current information about the inside of the head, e.g. the brain, without unnecessary dose exposure. The current scan can thus be performed even without low dose pre-scans to find a suitable trajectory. As an example, the current scan can be required after opening up a part of the skull. In such scenario, brain-shift may occur: the brain repositions itself by elastic deformation. For very accurate procedures, a new, i.e. current 3D-scan will be required to have an image of this new situation. The computed trajectory provides the basis for this new 3D scan.

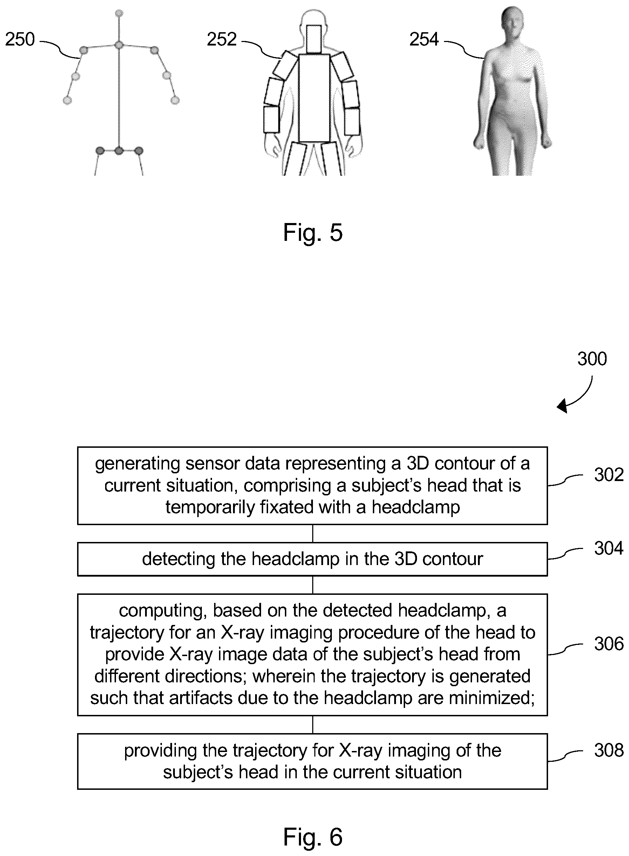

Fig. 5 shows three different approaches of subject recognition. On the left, a kinematic model 250 of a kinematic approach is shown. The kinematic approach defines the axes of the moving body parts and their spatial relation to each other. In the middle, a planar model 252 of a planar approach is shown. The planar approach divides the moving body parts into planar segments and determines their relation to each other. Further, on the right, a volumetric model 254 of a volumetric approach is shown. The volumetric approach detects the actual surface the body and defines a respective volume.

Fig. 6 shows basic steps of an example of a method 300 for acquiring X-ray images during human brain related interventions. The method 300 comprises the following steps:
- In a first step 302, sensor data is generated representing a 3D contour of a current situation, comprising a subject's head that is temporarily fixated with a headclamp.
- In a second step 304, the headclamp is detected in the 3D contour.
- In a third step 306, based on the detected headclamp, a trajectory is computed for an X-ray imaging procedure of the head to provide X-ray image data of the subject's head from different directions; the trajectory is generated such that artifacts due to the headclamp are minimized.
- In a fourth step 308, the trajectory is provided for X-ray imaging of the subject's head in the current situation.

Examples for neurosurgery procedures with head-clamp are: thermal and cryoablation procedures, deep brain stimulation, direct embolization, hematoma evacuation, cerebrospinal fluid diversion, aneurysm treatment, e.g. clipping, arteriovenous fistula (AVF) or arteriovenous malformation (AVM) treatment, tumor resection, endoscopic intraventricular tumor resection, skull base and transsphenoidal procedures, e.g. entering the subject's body via the nose.

In an option of Fig. 6, but not shown in detail, it is provided:
- Clamping a subject's head in an initial position with a headclamp;
- Scanning the current situation to generate the sensor data representing the 3D contour;
- Simulating an X-ray scan and assessing the simulation results in terms of artifacts;
- Providing at least one proposal for a change of the headclamp position to minimize simulated artifacts;
- Selecting one of the at least one proposals for change and readjusting the headclamp;
- Scanning the resulting situation and computing an updated trajectory; and
- Providing the updated trajectory for acquiring a plurality of images.

In an example, instead of the second scanning and computing, the trajectory is updated based on the proposal for the change of the headclamp position.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A control device (10) for acquiring X-ray images during human brain related interventions, the control device comprising:
- a data input (12);
- a data processor (14); and
- an output interface (16);
wherein the data input is configured: to provide sensor data (18) representing a 3D contour of a current situation, comprising a subject's head that is temporarily fixated with a headclamp;
wherein the data processor is configured: to detect the headclamp in the 3D contour; and to compute a trajectory (20) for an X-ray imaging procedure of the head to provide X-ray image data comprising a sequence of at least two images of the subject's head from different directions; wherein the data processor is configured to generate the trajectory such that artifacts due to the headclamp are minimized; and
wherein the output interface is configured: to provide the trajectory for X-ray imaging of the subject's head in the current situation.

2. Device according to claim 1, wherein the data processor is configured to: detect the subject's head in the 3D contour; and to compute the trajectory also based on the detected subject's head.

3. Device according to claim 1 or 2, wherein the data processor is configured to: detect further body parts of the subject in the 3D contour that might interfere with X-ray imaging; and to compute the trajectory also based on the detected further body parts of the subject.

4. Device according to claim 1, 2 or 3, wherein, for detecting the subject's head or body parts or headclamp parts, the data processor is configured to provide one of the group comprising image recognition, contour reconstruction and pose tracking.

5. Device according to one of the preceding claims, wherein, for computing the trajectory, the data processor is configured to consider different views having a common volume that contains the region of interest.

6. Device according to one of the preceding claims, wherein, for computing the trajectory, the data processor is configured to simulate possible changes for the arrangement of the headclamp and to consider the changes for computing alternative trajectories; and
wherein the data processor is configured to compare the alternative trajectories and to select at least one and to provide the respective selection as proposal for a change of the headclamp.

7. A medical X-ray imaging system (100; 200), comprising:
- an X-ray imaging device (102; 202) with a movable X-ray source (104; 204) and a movable X-ray detector (106; 206);
- a control device (10) according to one of the preceding claims; and
- a sensor arrangement (108; 208);
wherein the sensor arrangement is configured to provide the sensor data representing a 3D contour of a current situation; and
wherein the X-ray imaging device is configured to provide X-ray image data of a subject's head from different directions along the trajectory generated by the control device.

8. System according to claim 7, wherein the sensor arrangement comprises at least one sensor attached to the X-ray source or to the X-ray detector.

9. System according to claim 7 or 8, wherein the at least one sensor is:
i) an optical camera;
ii) a distance camera;
iii) a thermal camera;
iv) a hyperspectral camera; or
v) a Lidar sensor.

10. System according to claim 7, 8 or 9, wherein the X-ray imaging device is configured to perform a scanning movement with the sensor arrangement to provide a sensor scan of the current situation.

11. System according to one of claims 7 to 10, wherein the X-ray imaging device comprises a movable holding structure for the X-ray detector, and a separate movable holding structure for the X-ray source; and
wherein, preferably, the movable holding structure for the X-ray detector comprises a detector robot, and the movable holding structure for the X-ray source comprises a source robot.

12. System according to one of claims 7 to 11, wherein the sensor arrangement is configured to detect material properties of objects.

13. A method (300) for acquiring X-ray images during human brain related interventions, the method comprising the following steps:
- generating (302) sensor data representing a 3D contour of a current situation, comprising a subject's head that is temporarily fixated with a headclamp;
- detecting (304) the headclamp in the 3D contour;
- computing (306), based on the detected headclamp, a trajectory for an X-ray imaging procedure of the head to provide X-ray image data of the subject's head from different directions; wherein the trajectory is generated such that artifacts due to the headclamp are minimized; and
- providing (308) the trajectory for X-ray imaging of the subject's head in the current situation.

14. Method according to claim 13, wherein it is provided:
- clamping a subject's head in an initial position with a headclamp;
- scanning the current situation to generate the sensor data representing the 3D contour;
- simulating an X-ray scan and assessing the simulation results in terms of artifacts;
- providing at least one proposal for a change of the headclamp position to minimize simulated artifacts;
- selecting one of the at least one proposals for change and readjusting the headclamp;
- scanning the resulting situation and computing an updated trajectory; and
- providing the updated trajectory for acquiring a plurality of images.

15. Computer program comprising instructions which, when the program is executed by a computer, cause the computer to carry out the method of claim 13 or **14.**
